# EUROPÄISCHE PATENTSCHRIFT

(11) **EP 1 214 447 B1**
(45) Veröffentlichungstag und Bekanntmachung des Hinweises auf die Patenterteilung: **14.09.2005**
(21) Anmeldenummer: 00967757.6
(22) Anmeldetag: 22.09.2000
(51) Int. Cl.: C12Q 1/48, G01N 33/574

(54) **NACHWEIS DES PYRUVATKINASE-ISOENZYMS IM STUHL**
DETECTING THE PRESENCE OF THE PYRUVATE KINASE ISOENZYME IN FECES
DETERMINATION DE LA PRESENCE D'ISOENZYMES DE LA PYRUVATE KINASE DANS LES SELLES

(30) Priorität: 24.09.1999 DE 19945947
(43) Veröffentlichungstag der Anmeldung: 19.06.2002
(73) Patentinhaber: Schebo Biotech AG, 35394 Giessen (DE)
(72) Erfinder: EIGENBRODT, Erich, 35440 Linden (DE); SCHEEFERS-BORCHEL, Ursula, 35435 Wettenberg (DE); SCHEEFERS, Hans, 35435 Wettenberg (DE)
(74) Vertreter: Dey, Michael
(86) Internationale Anmeldenummer: PCT/EP2000/009303
(87) Internationale Veröffentlichungsnummer: WO 2001/021826

(56) Entgegenhaltungen:
- WO-A-97/09600
- DE-A- 3 838 900
- OREMEK G M ET AL: "The pyruvate kinase isoenzyme tumor M2 (Tu M2-PK) as a tumor marker for renal carcinoma." ANTICANCER RESEARCH, Bd. 19, Nr. 4A, Juli 1999 (1999-07), Seiten 2599-2602, XP000991276 ISSN: 0250-7005 in der Anmeldung erwähnt
- EIGENBRODT E ET AL: "Quantification of tumor type M2 pyruvate kinase (Tu M2-PK) in human carcinomas." ANTICANCER RESEARCH, Bd. 17, Nr. 4B, 1997, Seiten 3153-3156, XP000991280 ISSN: 0250-7005 in der Anmeldung erwähnt

## Beschreibung

Die vorliegende Erfindung betrifft ein Verfahren zum selektiven, qualitativen oder/und quantitativen Nachweis des Pyruvatkinase-Isoenzyms vom Typ Tumor M2 (Tumor M2-PK), welches als Tumormarker dient, im menschlichen und tierischen Stuhl zum Nachweis eines malignen Geschehens im Gastrointestinaltrakt und der Bauchhöhle (Speiseröhre, Magen, Dünndarm, Bauchspeicheldrüse und Dickdarm).

WO 97/09600 beschreibt ein Verfahren zur Isolierung von Zellen aus Stuhl und die Analyse der in den Zellen enthaltenen Proteine. Mit diesem Verfahren ist lediglich der Nachweis von abgeblätterten Gastrointestinalepithelzellen möglich. Tumore, welche keine Zellen abgeben, beispielsweise feste Tumore, können mit einem solchen Verfahren nicht erkannt werden.

Organ-spezifische Isoenzyme, welche während des Metastasierungsprozesses innerhalb eines Tumors verbleiben, sind exzellente Indikatoren für die Malignität (Tumormarker). Isoenzyme des Kohlenhydratstoffwechsels, insbesondere Pyruvatkinase-Isoenzyme und vor allem die Pyruvatkinase vom Typ Tumor M2-PK, scheinen solche Indikatoren zu sein.

Pyruvatkinase (PK)-Isoenzyme (E.C.2.7.1.40) existieren in vier Unterarten: L, R, M1 und M2. Untersuchungen haben gezeigt, daß maligne Tumoren aus menschlichem oder tierischem Ursprung eine besondere Art von Pyruvatkinase vom Typ M2 exprimieren, die sogenannte Tumor M2-PK, und zwar unabhängig von ihrer Herkunft (Eigenbrodt et al., Critical Reviews in Oncogenesis, 3 (1, 2) (1992) 91-115).

Alle bisher untersuchten Tumoren enthielten dieses spezielle Isoenzym. Im Gegensatz zu anderen Pyruvatkinasen, die vorwiegend in einer tetrameren Form vorliegen, tritt Tumor M2-PK in dimerer oder monomerer Form auf und ist an Serin und Tyrosin durch oncogen-kodierte Kinase phosphoryliert (Eigenbrodt et al., (1997) Anticancer Research, 17:3153-3156, Eigenbrodt et al., (1 998) Anticancer Research 18:3267-3274, Steinberg et al., (1999) virchows Arch 434: 213-220).

Die Umwandlung von der tetrameren Form des Isoenzyms in die monomere und/oder dimere Form scheint einen wesentlichen Schritt in der Tumorentstehung darzustellen. Dieses spezielle Tumor M2-PK Isoenzym wird von den Tumorzellen freigesetzt und ist in Körperflüssigkeiten quantitativ nachweisbar. Die Konzentration des Tumor M2-PK Isoenzyms korreliert mit der Malignität der Tumoren. Tumor M2-PK ist daher als Tumormarker anzusehen.

Es wurden bereits Antikörper entwickelt, die spezifisch für Tumor M2-PK sind und die nicht mit andern Pyruvatkinase-Isoenzymen vom Typ L, R, M1 oder M2 (normal M2) kreuzreagieren. Mit Hilfe dieser Antikörper konnte ein sensitiver Immunoassay entwickelt werden, mit dem Tumor M2-PK in Seren oder Plasma von Tumorpatienten nachgewiesen werden konnte (Scheefers-Borchel et al., (1994), Research-trends. R. Klapdor (ed.), W. Zuckschwerdt Verlag GmbH, München).

Bislang wurde dieser qualitative und quantitative Nachweis der Pyruvatkinase vom Typ Tumor M2 (Tumor M2-PK) im Serum oder Plasma mittels eines ELISA's durchgeführt, wie er u.a. in EP 0 444 118 beschrieben ist.

Dieser Test zeigt sehr gute Sensitivitäten und Spezifitäten bei einer Vielzahl von Tumorerkrankungen (Brinck, U., Eigenbrodt, E., Oehmke, M., Mazurek, S., Fischer, G. (1994) L- and M2-Pyruvate Kinase Expression in Renal Cell Carcinomas and their Metastases, Virch. Arch., 424: 177-185; Cerwenka, H., Aigner, R., Bacher, H., Werkgartner, G., El-Shabrawi, A., Quehenberger, F., Mischinger, HJ. (1999) TUM2-PK (Pyruvate Kinase Type Tumor M2), CA19-9 and CEA in Patients with Benign, Malignant and Metastasizing Pancreatic Lesions, Anticancer Research 19: 849-852; Eigenbrodt, E., Mazurek, S., Friis, R.R. (1998) Double role of pyruvatkinase type M2 in the regulation of phosphometabolite pools, Cell growth and Oncogenesis 15; Hugo,F., Fischer, G., Eigenbrodt, E. (1999) Quantitative Detection of Tumor M2-PK in Serum and Plasma, Anticancer Research (1999), 19:2753-2758; Mazurek, S., Grimm, H., Wilker, S., Leib, S., Eigenbrodt, E. (1998) Metabolic Characteristics of Different Malignant Cancer Cell Lines, Anticancer Research 18: 3275-32821; Oremek, G.M., Eigenbrodt, E., Rädle, J., Zeuzem, St., Seiffert, U.B. (1997) Value of the Serum Levels of the Tumor Marker Tu M2-PK in Pancreatic Cancer, Anticancer Research, 17: 3031-3034 Oremek, G.M., Teigelkamp, S., Kramer, W., Eigenbrodt E., Usadel, K.-H. (1999) The Pyruvate Kinase Isoenzyme Tumor M2 (Tumor M2-PK) as a Tumor Marker for Renal Carcinoma, Anticancer Research (1999), 19: 2599-2602; Scheefers-Borchel, U., Scheefers, H., Michel, Will, A., Fischer, G., Basenau, D., Dahlmann, N., Laumen, R., Mazurek, S., Eigenbrodt E. (1994) Quantitative determination (ELISA) of pyruvatkinase type tumor M2, in: Current Tumor Diagnosis, Applications, Clinical Relevance, Research-trends. R. Klapdor (ed.), W. Zuckschwerdt Verlag GmbH, München; Wechsel, H.W., Petri, E., Feil G., Bichler, K.-H. (1997) Tumor Specific Pyruvate Kinase (Tumor M2-PK): A potential marker for renal cell carcinoma (RCC)1, J Urology 157: 424; Wechsel, H.W., Petri, E., Bichler, K.-H., Feil G. (1999) Marker for Renal Cell Carcinoma (RCC): The Dimeric Form of Pyruvate Kinase Type M2 (Tumor M2-PK), Anticancer Research (1999), 19: 2583-2590.

Die Definition und Einteilung von Tumormarkern wird wie folgt vorgenommen:

Tumormarker sind in Blut, Serum oder anderen Körperflüssigkeiten auftretende Makromoleküle oder Antigene, deren Konzentrationsänderungen in gewisser Beziehung mit der Entstehung und dem Wachstum von malignen Tumoren eines Individuums stehen.

### Kriterien für Tumormarker

Der "ideale Tumormarker" sollte folgende Eigenschaften haben:
- hohe Spezifität, d.h. bei benignen Erkrankungen und gesunden Personen nicht nachweisbar sein;
- hohe Sensitivität, d.h. er kann in einem hohen Prozentsatz der Tumorpatienten nachgewiesen werden;
- Organspezifität;
- gute Korrelation mit den Tumorstadien bzw. der Tumormasse;
- gute Aussagekraft zur Malignität;
- Beziehung zur Prognose;
- verlässliche Vorhersagewerte.

Die Kriterien der 100 %igen Spezifität und der 100 %igen Sensitivität werden bis heute von keinem der bekannten Tumormarker erfüllt.

Da jedoch die Tumor M2-PK bei allen Tumorerkrankungen im Serum oder Blutplasma von Patienten nachgewiesen werden kann, sind Aussagen über die Art und die Lokalisation des betroffenen Organs oder Gewebes im Körper des Patienten nicht möglich. Des Weiteren wäre es wünschenswert, möglichst frühzeitig ein tumuröses Geschehen im Gastrointestinaltrakt, insbesondere in einer Wachstumsphase, in der der Tumor noch keinen Kontakt mit dem Gefäßsystems des Körpers aufgenommen hat (in der "Polyp-cancer-sequence", zeitlich vor der Infiltration der Submukosa), nachweisen zu können.

Beim Verdacht auf ein neoplastisches Geschehen im Gastrointestinaltrakt, besonders im Hinblick auf die sogenannte Adenom-Carcinom-Sequenz bei Polypen (Polyposis coli), wird nach dem Stand der Technik mittels verschiedener Bestimmungsmethoden versucht, occultes Blut im Stuhl nachzuweisen. Hierzu werden nicht-immunologische Tests (z.B. Pseudoperoxidase-Aktivität, Porphyrin-Nachweis) und immunologische Test verwendet.

Beide Testprinzipien sind jedoch nicht sehr spezifisch, da sie durch eine Vielzahl von Einflußgrößen gestört werden können (falsch positiv/falsch negativ, z.B. durch Nichteinhalten dringend notwendiger Diätvorschriften von Seiten des Patienten und von einer Reihe von Arzneimitteln sowie durch überhöhte Vitamin-C-Gabe (z.B. in Gemüse, Fruchtsäften etc., Thomas, L., Labor und Diagnose, 5. Auflage, 1998).

Ein positiver Test auf occultes Blut im Stuhl muss so lange abgeklärt werden, bis die Blutungsquelle lokalisiert ist bzw. die Ursache der Blutung gefunden wurde. Der klinische Befund rechtfertigt in jedem Fall eine zügig durchzuführende weitergehende Diagnostik, z.B. durch Endoskopie, Sonographie, Röntgen. Das Vorliegen von Blut im Stuhl ist aber nicht immer auf ein tumoröses Geschehen zurückzuführen und andersherum bedeutet das Fehlen von Blut im Stuhl auch nicht sicher, dass kein Tumor im Darm vorhanden ist.

Die Erfindung hat damit zum Ziel, den weiterhin bestehenden Bedarf an einem spezifischen, leicht durchzuführenden Verfahren zu befriedigen, sodass ein neoplastisches Geschehen, vor allem im Hinblick auf die Problematik der sogenannten Adenom-Carcinom-Sequenz bei Polypen (Polyposis coli) im Gastrointestinaltrakt, besonders frühzeitig und zweifelsfrei nachgewiesen werden kann.

Diese Aufgabe wird erfindungsgemäß gelöst durch ein Verfahren zum selektiven, qualitativen oder/und quantitativen Nachweis des Pyruvatkinase-Isoenzyms vom Typ Tumor M2 (Tumor M2-PK), welches als Tumor-Marker dient, im menschlichen und tierischen Stuhl zum Nachweis eines malignen Geschehens im Gastrointestinaltrakt, welches dadurch gekennzeichnet ist, dass man die Tumor M2-PK mit Hilfe mindestens eines Rezeptors, insbesondere eines Antikörpers, der spezifisch Tumor M2-PK bindet und mit keinem anderen Pyruvatkinase-Isoenzym kreuzreagiert nach dem Prinzip des Immunoassays in einer Stuhlprobe bestimmt.

Es wurde nämlich gefunden, daß sich der Gehalt an Tumor M2-PK direkt im Stuhl von Tumorpatienten nachweisen lässt, wobei der Gehalt direkt proportional zum Grad des malignen Geschehens im Gastrointestinaltrakt ist.

Dies ist umso überraschender, weil umfangreiche Untersuchung bezüglich anderer spezifischer Proteine im Stuhl keine Hinweise auf ihre Verwendung als Tumormarker zuließen.

Sowohl die Struktur als auch die physiko-chemischen Eigenschaften der Tumor M2-PK werden überraschenderweise nicht durch die erhebliche proteolytische Aktivität und die extremen physiologischen Gegebenheiten (z.B. pH-Wert, sauer im Magen, alkalisch im Darm) des Gastrointestinaltraktes nachhaltig beeinträchtigt. Dies gilt auch für den Nachweis der Tumor M2-PK mittels immunologischer Methoden.

Erfindungsgemäß wurde nämlich gezeigt, dass sich trotz der oben beschrieben Proteindenaturierung und Proteinverdauung im Gastrointestinaltrakt ein spezifischer Nachweis von Tumor M2-PK im Stuhl von Tumorpatienten durchführen lässt. Bislang wurde die Tumor M2-PK ausschließlich in nativen, nicht verdauten Patientenproben (Körperflüssigkeiten, Sera, Blutplasma) quantitativ bestimmt und diese Bestimmung in der klinischen Tumordiagnostik für eine Vielzahl von Tumorerkrankungen eingesetzt.

Überraschenderweise wurde nun aber festgestellt, dass Tumor M2-PK quantitativ auch bei intensiv homogenisierten, länger gelagerten Stuhlproben (zum Beispiel beim Probenversand) nachweisbar bleibt. Auch bei starker Verdünnung des Stuhles wird eine deutliche Reaktion erhalten.

Überraschenderweise wurde weiterhin festgestellt, daß der Nachweis auch ohne vorherige Extraktion in der Stuhlprobe selektiv erfolgt. Vorzugsweise ist jedoch ein Extraktionsverfahren unter vorzugsweiser Verwendung eines speziellen Detergens (CHAPS) zu verwenden (das Extraktionsverfahren wird näher in Beispiel 2 schrieben).

In einer bevorzugten Ausführungsform der Erfindung verwendet man als Antikörper einen monoklonalen Antikörper, der aus der Hybridomazelllinie Klon 1 (hinterlegt bei der Deutschen Sammlung für Mikroorganismen unter der Nummer DSM ACC 2155) erhältlich ist, oder aber einen Antikörper verwendet, der gleiche Spezifität und Selektivität aufweist. Derartige Antikörper können beispielsweise wie in der EP 0 444 118 beschrieben, hergestellt werden. Die Herstellung ist allerdings nicht kritisch, da dem Fachmann Verfahren zur Herstellung von spezifischen monoklonalen Antikörpern gut bekannt sind. Es wird hierzu das Antigen, im vorliegenden Fall also die Tumor M2-PK in in üblicher Weise gereinigter Form (oder Teilstücke derselben) zur Erzeugung von Antikörpern verwendet. Prinzipiell kann hierzu das Verfahren, welches erstmals von Köhler und Milstein beschrieben wurde, angewandt werden, wobei dem Fachmann auch Abwandlungen und Weiterentwicklungen dieser Verfahren bekannt sind. Die Herstellung ist solange nicht kritisch, wie spezifische Antikörper erhalten werden, was durch Selektion festgestellt werden kann. Die Selektion erfolgt auf Antikörper, welche spezifisch die Tumor M2-PK, jedoch nicht eines der anderen Isoenzyme der Pyruvatkinase, und auch nicht die Nichttumor M2-PK-Form binden.

Im erfindungsgemäßen Verfahren werden vorzugsweise folgende Schritte durchgeführt:
(a) Man bringt die Probe mit mindestens zwei verschiedenen Rezeptoren in Kontakt, von denen der erste Rezeptor R1 in fester Phase vorliegt und mit Tumor M2-PK bindefähig ist, und der zweite Rezeptor R2 in flüssiger Phase vorliegt, und ebenfalls mit Tumor M2-PK bindefähig ist, wobei Rezeptor R2 eine Markierung trägt oder die Bindung an ein detektierbares Molekül vermittelt;
(b) man trennt die feste von der flüssigen Phase;
(c) man bestimmt die Markierung oder das detektierbare Molekül in der festen Phase nach an sich bekannten Methoden und quantifiziert entsprechend die Menge an in der Probe vorhandener Tumor M2-PK, wobei man erfindungsgemäß als mindestens einen der Rezeptoren 1 oder 2 einen Antikörper verwendet, der spezifisch mit Tumor M2-PK bindefähig ist und zwischen anderen Pyruvatkinas-Isoenzymen diskriminiert, d.h. keines der anderen Isoenzyme und auch nicht die Nichttumor M2-PK-Form bindet.

Obwohl es selbstverständlich auch möglich ist, zwei Antikörper zu verwenden, welche beide spezifisch mit Tumor M2-PK nach obiger Definition bindefähig sind, reicht es für den zweiten der Rezeptoren 1 oder 2 in der Regel aus, einen nicht so spezifischen Antikörper zu verwenden. In dem Fall, in dem als Rezeptor R1 bereits ein spezifisch nur mit Tumor M2-PK bindefähiger Antikörper verwendet wurde, liegt nach Abtrennung der flüssigen Phase nur die Tumor M2-PK an diesen Rezeptor gebunden vor. Der Nachweisrezeptor R2 muss daher lediglich mit Tumor M2-PK bindefähig sein, muss aber nicht unbedingt eine Diskriminierung zwischen PK-Isoenzymen erlauben.

In dem Fall, in dem als Rezpetor R1 ein weniger spezifischer Antikörper eingesetzt wird, wird ein Rezeptor R2 verwendet, der spezifisch nur mit der Tumor M2-PK bindefähig ist, sodass ein detektierbares Signal nur bei Vorhandensein von Tumor M2-PK entsteht.

Wie oben bereits erwähnt, können jedoch auch zwei spezifisch nur mit Tumor M2-PK bindefähige Antikörper eingesetzt werden, um womöglich eine noch höhere Spezifität zu erreichen.

In einer bevorzugten Ausführungsform des erfindungsgemäßen Verfahrens verwendet man als Rezeptor R1 einen spezifisch mit Tumor M2-PK bindefähigen und zwischen anderen Pyruvatkinase-Isoenzymen diskriminierenden Antikörper.

In einer weiteren bevorzugten Ausführungsform verwendet man als Rezpetor R2 einen Antikörper, der selbst eine Markierung trägt. Damit ist ein direkter Nachweis noch schneller möglich als in dem Fall, wo ein detektierbares Molekül an Rezeptor R2 gebunden ist. Eine weitere bevorzugte Ausführungsform der Erfindung ist ein Verfahren, bei dem man als Rezeptor R2 einen enzymgebundenen Antikörper verwendet und das Verfahren als Enzyme Linked Immuno Sorbent Assay (ELISA) ausführt.

Zur Durchführung des erfindungsgemäßen Verfahrens eignet sich z.B. ein Testkit zur Diagnose eines malignen Tumorgeschehens im Gastrointestinaltrakt und insbesondere im Darm, der mindestens einen Rezeptor für Tumor M2-PK enthält, der mit keinem anderen Pyruvatkinase-Isoenzym kreuzreagiert, sowie gegebenenfalls weitere für die Durchführung eines Monoassays notwendige Reagenzien.

Der Testkit ist insbesondere für die Durchführung des erfindungsgemäßen Verfahrens ausgestaltet. Deshalb enthält der Testkit vorzugsweise einen zweiten Rezeptor R2, der eine Markierung trägt oder die Bindung an ein detektierbares Molekül vermittelt.

Vorzugsweise wird ein an Tumor M2-PK spezifisch bindender Antikörper mittels bekannten Verfahren an eine Festphase immobilisiert. Diese Festphase ist in einer bevorzugten Ausgestaltung der Erfindung ebenfalls Bestandteil des Testkits. Die zu untersuchende Probe wird dann mit dem vorzugsweise gelösten Protein in Kontakt gebracht und in einem geeigneten Puffersystem mittels der Antikörper an die Festphase gebunden. Nach Waschen des so erhaltenen immobilisierten Antikörper-Tumor M2-PK-Komplexes wird dann ein weiterer markierter, z.B. mit Biotin versehener zweiter Antikörper zugesetzt, der an ein anderes Epitop des Tumor M2-PK bindet. Dann wird die Markierung bestimmt, z.B. mit Hilfe von Streptavidin-Peroxidase. Die Menge an gebundenem Marker ist direkt proportional der Menge an Tumor M2-PK in der Probe. Zweckmäßigerweise enthält der Testkit Referenzmaterial bekannten Gehalts an Tumor M2-PK zur quantitiven Bestimmung.

Selbstverständlich können auch alle anderen Arten von immunologischen Nachweisverfahren, welche mit Hilfe von Antikörpern durchgeführt werden, erfindungsgemäß eingesetzt werden. Gerade auch für die tatsächliche Formulierung als Testkit sind dem Fachmann viele Alternativen bekannt, die allesamt prinzipiell für die Durchführung des erfindungsgemäßen Verfahrens geeignet sind. So kann auch beispielsweise ein Test in Form eines Teststreifens konzipiert werden, auf dem in unterschiedlichen Zonen des Teststreifens die benötigten Antikörper entweder in löslicher Form oder festphasenfixiert angeordnet sind. Die Probe bzw. der Flüssigkeitsanteil der Probe oder ein Extrakt können dann den Teststreifen durchwandern und an der Detektionsstelle ein Signal liefern, wenn Tumor M2-PK in der Probe vorhanden ist. Die genaue Anordnung der einzelnen Komponenten auf dem Teststreifen ist abhängig vom angewandten immunologischen Verfahren und vom Fachmann leicht realisierbar.

Im Rahmen der vorliegenden Erfindung soll der Ausdruck "Rezeptor" bzw. "Antikörper" auch solche Teile oder Fragmente von Rezeptoren bzw. Antikörpern umfassen, welche die notwendige Bindung an Tumor M2-PK noch vermitteln. Es ist hierbei auch möglich, anstelle eines einzelnen Antikörpers ein Konjugat aus zwei Antikörpern einzusetzen. Beispielsweise kann man sich vorstellen, als Rezeptor R2 einen mit Tumor M2-PK bindefähigen Antikörper zu verwenden und den Nachweis über einen weiteren markierten Antikörper zu führen, welcher gerichtet ist gegen den Fc-Teil des Rezeptors 2 und eine Markierung trägt oder wiederum an ein detektierbares Molekül gekoppelt ist. Diese Konjugatbildung aus zwei Antikörpern soll im Rahmen der vorliegenden Erfindung mitumfasst sein, wenn R2 so definiert ist, dass er die Bindung an ein detektierbares Molekül vermittelt. Analoges gilt für die Bindung des Rezeptors R1 an die feste Phase. Auch diese Bindung kann über Festphasen-gekoppelte Antikörper erfolgen, an welche der Fc-Teil des Rezeptors 1 bindet.

Viele weitere Ausgestaltungen der Erfindung sind denkbar und für den Fachmann ohne Weiteres durchführbar. Diese Ausgestaltungen sind daher im Rahmen der vorliegenden Erfindung mitumfasst, solange sie den Nachweis der Tumor M2-PK in einer Stuhlprobe ermöglichen.

In besonders bevorzugten Ausführungsformen der Erfindung enthält der Testkit als einen der Rezeptoren den monoklonalen Antikörper, der aus der Hybridomzelllinie Klon 1 (DSM ACC 2155) erhältlich ist. In einer weiteren bevorzugten Ausführungsform wird ein an die Festphase gebundener, für Tumor M2-PK spezifischer Antikörper verwendet, der nicht mit anderen Isoenzymen oder der Nichttumor M2-PK kreuzreagiert.

Bevorzugt ist der lösliche Rezeptor R2 ein markierter Antikörper.

Erfindungsgemäß ist es auch möglich, die Analytkonzentration mittels Biosensoren zu bestimmen, wie z.B. amperometrische Sensoren, potentiometrische, ionenselektive potentiometrische oder photometrische Sensoren oder auch solche mittels Halbleiterelektroden wie Feldeffekttransistoren (FET), chemosensitive Feldeffekttransistoren (CHEMFET), suspended-gate-Feldeffekttransistoren (SGFET) oder ionensensitiven Feldeffekttransistoren. Derartige Biosensoren sind zusammenfassend in E.A. H. Hall und G. Hummel in "Biosensoren", Springer Verlag Heidelberg, Deutschland, 1995 beschrieben. Weitere Entwicklungen von ionensensitiven Feldeffekttransistoren (ISFET) oder optischen Detektoren sind unter anderem von F. Aberl und H. Wolf in "Aktuelle Trends in der Immunsensorik", Labor 2000, S. 70-96 (1993) beschrieben. Ebenfalls geeignet ist das erfindungsgemäße Verfahren für die Durchführung mittels piezoelektrischen Schwingquarzen und Oberflächenwellenelementen, welche als Mikrowaagen verwendet werden können. Dabei wird der primäre Antikörper (der sog. Catcher) auf einem piezoelektrischen Substrat immobilisiert und nach Bindung mit der zu analysierenden Tumor M2-PK eine Änderung der Schwingungsfrequenz des Quarzes gemessen. Derartige Sensoren sind beispielsweise von A. Leidl et al. In "Proceedings of the Second International Symposium on Minaturized Total Analyses Systems µTAS", Basel 1 996, beschrieben. Quarzkristallmikrowaagen, wie sie von C. Köslinger et al., Fresenius J. Anal. Chem (1994), 349: 349-354, beschrieben sind, haben sich als besonders geeignet erwiesen.

Signifikante Verbesserungen der Immunoassays hinsichtlich Sensitivität, Testkinetik und weiteren Punkten wie dynamic range and format flexibility können durch Anwendung der Elektrochemilumineszenz erreicht werden. Elektrochemilumineszenz ist der Vorgang, durch den Licht erzeugt wird, wenn eine geringe Spannung an eine Elektrode angelegt wird, wodurch eine cyclische Redox-Reaktion in einem Ruthenium Metall Ion getriggert wird (Bruno, G. (1997) Rec.Rp pp. 175-179; Williams R. (1996), Amer. Biotech., p. 26).

Die erfindungsgemäß zu verwendenden Antikörper sind, wie oben beschrieben, auf an sich bekannte Weise erhältlich. Vorzugsweise wird zuerst Tumor M2-PK, z.B. aus Dickdarmtumoren isoliert (durch Anwendung der Chromatographie an DEAE Sephadex A-50 und anschließender Affinitätschromatogrophie an blauer Sepharose CL 6B), wie bei K. Scheiner-Bobis beschrieben (Promotionsarbeit an der Justus Liebig-Universität in Gießen, eingereicht am 8. November 1988). Danach wird ein Versuchstier mit der so erhaltenen Tumor M2-PK bzw. mit Bruckstücken davon, welche die entsprechenden Epitope aufweisen, immunisiert und die gebildeten Antikörper isoliert. Derartige Bruchstücke, welche zur Immunisierung verwendet werden, können z.B. aus einer Protease-Verdauung der gereinigten Tumor M2-PK stammen oder aus synthetischen Teilpeptiden derselben bestehen. Die Herstellung solcher Teilpeptide ist dem Fachmann an sich bekannt. Es werden dabei aus der Sequenz z.B. mit Hilfe von Computerprogrammen Sequenzen ausgesucht, welche entsprechende Epitope enthalten können.

Diese Sequenzen werden dann auf ihre Verwendbarkeit zur Herstellung spezifischer Antikörper getestet.

Vorzugsweise werden nach der Methode von Köhler, Milstein (Natu-re 256, 495-497 (1975) erhältliche monoklonale Antikörper verwendet. Dabei werden beispielsweise BALB/C-Mäuse mit isolierter Tumor M2-PK immunisiert und die Milzzellen dieser Tiere mit einer Myelomazelllinie, beispielsweise PA 1, fusioniert. Die in den Ascites sezernierten Antikörper werden, beispielsweise im ELISA oder RIA, auf ihre Spezifität getestet und isoliert. Üblicherweise gehören die so erhaltenen Antikörper zur Klasse IgG1.

Die Erfindung wird durch die folgenden Beispiele weiter erläutert:

### Beispiel 1

### Einwiegen der Stuhlproben

Ein etwa 12 ml umfassendes Einwegröhrchen und eine mikrobiologische Impföse (z.B. Sarstedt) werden mit einer empfindlichen digitalen Laborwaage auf 0 austariert. Anschließend wird mit der Impföse der Stuhl eingewogen, indem man mit der Öse in die Stuhlprobe sticht und die an der Spitze verbleibende Stuhlmenge (etwa 100 mg) in das Einwegröhrchen einbringt. An Stelle der Impföse kann auch ein Zahnstocher benutzt werden.

Entsprechend der gewogenen Stuhlprobenmasse werden die Volumina des zuzugebenden Extraktionspuffers variiert (z. B. 100 mg Stuhl + 10 ml Puffer oder 75 mg Stuhl + 7,5 ml Puffer). Endkonzentration: 10 mg Stuhl/ml Extraktionspuffer.

### Beispiel 2

### Homogenisation und Extraktion der Stuhlproben

Die Stuhlprobensuspension wird bei Raumtemperatur mehrfach kräftig mit einem Reaganzglas-Schüttelgerät (z.B. VORTEX) gemixt. Die Stühle müssen gut homogenisiert sein. Um eine vollständige Extraktion zu gewährleisten, ist es wichtig, dem phosphatgepufferten Extraktionspuffer das Detergenz CHAPS (3-[(3-Cholamidopropyl)dimethylammonio]-1-propansulfonat, 10 mM (Sigma) beizumischen.

### Beispiel 3

### ELISA

Eine ELISA-Platte wird mit einem monoklonalen Antikörper beschichtet, der nur Tumor M2-PK erkennt. Tumor M2-PK aus EDTA-Plasmaproben und aus Standards bindet an diesen Antikörper und wird dadurch an die Platte gebunden. Ein zweiter monoklonaler Antikörper, der biotinyliert ist, bindet im nächsten Inkubationsschritt an Tumor M2-PK. Nachfolgend wird ein Konjugat von POD (Peroxidas) und Streptavidin an die Biotineinheit des Antikörpers gebunden. Die Peroxidase oxidiert ABTS 2,2'-Azino-bis-(3-ethylbenzothiazolin-6-sulfonsäure) diammoniumsalz) unter Ausbildung einer dunklen grünen Farbe. Schließlich wird die Konzentration von oxidiertem ABTS fotometrisch bestimmt.

## Patentansprüche

1. Verfahren zum selektiven, qualitativen oder/und quantitativen Nachweis des Pyruvatkinase-Isoenzyms vom Typ Tumor M2 (Tumor M2-PK), welches als Tumor-Marker dient, im menschlichen und tierischen Stuhl zum Nachweis eines malignen Geschehens im Gastrointestinaltrakt,
**dadurch gekennzeichnet,**
**dass** man die Tumor M2-PK mit Hilfe mindestens eines Rezeptors, der mit keinem anderen Pyruvatkinase-Isoenzym kreuzreagiert nach dem Prinzip des Immunoassays in einer Stuhlprobe bestimmt.

2. Verfahren nach Anspruch 1 zum selektiven, qualitativen oder/und quantitativen Nachweis des Pyruvatkinase-Isoenzyms vom Typ Tumor M2 (Tumor M2-PK), welches als Tumor-Marker dient, im menschlichen und tierischen Stuhl zum Nachweis eines malignen Geschehens im Gastrointestinaltrakt,
**dadurch gekennzeichnet,**
**dass** man die Tumor M2-PK mit Hilfe mindestens eines Antikörpers, der spezifisch Tumor M2-PK bindet und mit keinem anderen Pyruvatkinase-Isoenzym kreuzreagiert nach dem Prinzip des Immunoassays in einer Stuhlprobe bestimmt.

3. Verfahren nach Anspruch 2
**dadurch gekennzeichnet,**
**dass** man als Antikörper einen monoklonalen Antikörper verwendet, der aus der Hybridoma-Zelllinie Klon 1 (Nr. DSM ACC 2155) erhältlich ist, oder einen Antikörper mit gleicher Spezifität und Selektivität.

4. Verfahren nach Anspruch 2 oder 3,
**dadurch gekennzeichnet,**
**dass** man
a) die Probe mit mindestens zwei verschiedenen Rezeptoren in Kontakt bringt, von denen der erste Rezeptor RI in fester Phase vorliegt und mit Tumor M2-PK bindefähig ist und der zweite Rezeptor R2 in flüssiger Phase vorliegt und ebenfalls mit Tumor M2-PK bindefähig ist und wobei Rezeptor R2 eine Markierung trägt oder die Bindung an ein detektierbares Molekül vermittelt,
b) die feste von der flüssigen Phase trennt,
c) die Markierung oder das detektierbare Moleküls in der festen Phase bestimmt und entsprechend die Menge an in der Probe vorhandener Tumor M2-PK quantifiziert, wobei man als mindestens einen der Rezeptoren 1 oder 2 einen Antikörper verwendet, der spezifisch mit Tumor M2-PK bindefähig ist und zwischen anderen Pyruvatkinase-Isoenzymen diskriminiert.

5. Verfahren nach Anspruch 4,
**dadurch gekennzeichnet,**
**dass** man den spezifisch mit Tumor M2-PK bindefähigen und zwischen anderen Pyruvatkinase-Isoenzymen diskriminierenden Antikörper als Rezeptor 1 einsetzt.

6. Verfahren nach einem der vorhergehenden Ansprüche,
**dadurch gekennzeichnet,**
**dass** man einen Antikörper als Rezeptor 2 verwendet, der eine Markierung trägt.

7. Verfahren nach einem der Ansprüche 1 bis 5,
**dadurch gekennzeichnet,**
**dass** man einen Enzym-gebundenen Antikörper als Rezeptor 2 verwendet und das Verfahren als ELISA durchführt.

## Claims

1. Method for the selective, qualitative or/and quantitative detection of the pyruvate kinase isoenzyme of the type tumour M2 (tumour M2-PK) which serves as a tumour marker, in human and animal faeces for detecting a malignant process in the gastrointestinal tract,
**characterized in that**
the tumour M2-PK is determined in a stool specimen by an immunoassay technique with the aid of at least one receptor which does not cross-react with any other pyruvate kinase isoenzyme.

2. Method as claimed in claim 1 for the selective, qualitative or/and quantitative detection of the pyruvate kinase isoenzyme of the type tumour M2 (tumour M2-PK) which serves as a tumour marker, in human and animal faeces for detecting a malignant process in the gastrointestinal tract,
**characterized in that**
the tumour M2-PK is determined in a stool specimen by an immunoassay technique with the aid of at least one antibody which specifically binds tumour M2-PK and does not cross-react with any other pyruvate kinase isoenzyme.

3. Method as claimed in claim 2,
**characterized in that**
a monoclonal antibody is used as the antibody which is obtainable from the hybridoma cell line clone 1 (No. DSM ACC 2155), or an antibody is used which has the same specificity and selectively.

4. Method as claimed in claim 2 or 3;
**characterized in that**
(a) the sample is contacted with at least two different receptors of which the first receptor R1 is present in a solid phase and is capable of binding to tumour M2-PK and the second receptor R2 is present in a liquid phase and is also capable of binding to tumour M2-PK, wherein receptor R2 carries a label or mediates binding to a detectable molecule;
(b) the solid phase is separated from the liquid phase;
(c) the label or the detectable molecule in the solid phase is determined and thus the amount of tumour M2-PK present in the sample is quantified, wherein an antibody is used as at least one of the receptors 1 or 2 which can specifically bind to tumour M2-PK and discriminates between other pyruvate kinase isoenzymes.

5. Method as claimed in claim 4,
**characterized in that**
the antibody capable of specifically binding to tumour M2-PK and able to discriminate between other pyruvate kinase isoenzymes is used as the receptor 1.

6. Method as claimed in one of the previous claims,
**characterized in that**
an antibody which carries a label is used as receptor 2.

7. Method as claimed in one of the claims 1 to 5,
**characterized in that**
an enzyme-bound antibody is used as receptor 2 and the method is carried out as an ELISA.

## Revendications

1. Procédé de détection sélective, qualitative ou/et quantitative de l'isoenzyme pyruvate kinase du type tumeur M2 (tumeur M2-PK), qui sert de marqueur de tumeur, dans des selles humaines et animales pour la détection d'un événement malin dans le tractus gastro-intestinal, **caractérisé en ce que** l'on identifie la tumeur M2-PK selon le principe de l'immuno-essai dans un échantillon de selles, à l'aide d'au moins un récepteur, qui ne présente pas de réaction croisée avec une autre isoenzyme pyruvate kinase.

2. Procédé selon la revendication 1 de détection sélective, qualitative ou/et quantitative de l'isoenzyme pyruvate kinase du type tumeur M2 (tumeur M2-PK), qui sert de marqueur de tumeur, dans des selles humaines et animales pour la détection d'un événement malin dans le tractus gastro-intestinal, **caractérisé en ce que** l'on identifie la tumeur M2-PK selon le principe de l'immuno-essai dans un échantillon de selles, à l'aide d'au moins un anticorps, qui se lie spécifiquement à la tumeur M2-PK et qui ne présente pas de réaction croisée avec une autre isoenzyme pyruvate kinase.

3. Procédé selon la revendication 2, **caractérisé en ce que** l'on utilise comme anticorps un anticorps monoclonal, que l'on peut obtenir à partir de la lignée cellulaire d'hybridome Klon 1 (n° DSM ACC 2155), ou un anticorps avec une spécificité et une sélectivité identiques.

4. Procédé selon la revendication 2 ou 3, **caractérisé en ce que** l'on
a) met en contact l'échantillon avec au moins deux récepteurs différents, parmi lesquels le premier récepteur R1 se trouve en phase solide et est capable de se lier avec la tumeur M2-PK et le deuxième récepteur R2 se présente en phase liquide et est susceptible de se lier également avec la tumeur M2-PK et dans lequel le récepteur R2 porte un marqueur ou permet la liaison à une molécule détectable,
b) on sépare la phase solide de la phase liquide,
c) on mesure le marqueur ou la molécule détectable en phase solide et on quantifie en correspondance la quantité de tumeur M2-PK présente dans l'échantillon, en utilisant comme au moins un des récepteurs 1 ou 2 un anticorps, qui est capable de se lier spécifiquement avec la tumeur M2-PK et qui discrimine entre d'autres isoenzymes pyruvate kinase.

5. Procédé selon la revendication 4, **caractérisé en ce que** l'on utilise comme récepteur 1 l'anticorps capable de se lier spécifiquement avec la tumeur M2-PK et discriminant entre d'autres isoenzymes pyruvate kinase.

6. Procédé selon l'une des revendications précédentes, **caractérisé en ce que** l'on utilise un anticorps comme récepteur 2, qui porte un marqueur.

7. Procédé selon l'une des revendications 1 à 5, **caractérisé en ce que** l'on utilise comme récepteur 2 un anticorps lié à une enzyme et l'on réalise le procédé sous forme ELISA.
